(19) Europäisches Patentamt
European Patent Office
Office européen des brevets

(11) Publication number: 0 269 299
A2

# (12) EUROPEAN PATENT APPLICATION

(21) Application number: 87309876.8

(22) Date of filing: 06.11.87

(51) Int. Cl.4: C07K 7/10 , A61K 37/02

(30) Priority: 07.11.86 DK 5319/86

(43) Date of publication of application:
01.06.88 Bulletin 88/22

(84) Designated Contracting States:
AT BE CH DE ES FR GB GR IT LI LU NL SE

(71) Applicant: NOVO INDUSTRI A/S
Novo Allé
DK-2880 Bagsvaerd(DK)

(72) Inventor: Faarup, Peter
15 Skraplanet
DK-3500 Vaerlose(DK)
Inventor: Johansen, Nils Langeland
45 Niels W. Gades Gade
DK-2100 Copenhagen(DK)
Inventor: Thogersen, Henning
26 Kaerbovaenge
DK-3520 Farum(DK)
Inventor: Lundt, Behrend Friedrich
118 Rosenhaven
DK-2980 Kokkedal(DK)
Inventor: Weis, Jan
6 Fuglsangvej
DK-2830 Virum(DK)

(74) Representative: Brown, John David et al
FORRESTER & BOEHMERT
Widenmayerstrasse 4/I
D-8000 München 22(DE)

## (54) Atrial peptides.

(57) Derivatives of atrial natriuretic peptides (ANP) wherein at least two of the amino acid residues in the ring structure (positions 7 - 23) are exchanged with other amino acid residues and/or which in the C terminal end (position 28) are elongated with Pro, Trp, Phe or N-MePhe or a polypeptide moiety having one of the last mentioned 4 amino acid residues in the N terminal end, have improved pharmacological properties.

EP 0 269 299 A2

## PEPTIDES

The present invention relates to novel synthetic atrial peptides having interesting pharmacological effects such as useful natriuretic, diuretic and vasodilating activity, and the use of such peptides as medicaments, for example in the treatment of hypertension, congestive heart failure and diseases in the renal function.

It is known from the pioneering observation (Life Science 28 (1981), 89 - 94), that extracts of atrial myocardium when injected intravenously into non-diurectic rats provoked a rapid and potent diuretic and natriuretic response. This natriuretic and diuretic factor of atrial origin has later on been purified both from the atrial muscle of the rat and from other mammals. The factor is now known to be a polypeptide which has been characterized in terms of the amino acid sequence. The polypeptide is composed of a 17 amino acid ring structure formed by a disulfide bridge between two cysteine residues and that ring structure is elongated at the amino and carboxy terminus to peptides of different length. Experimental evidence suggest that a 28 amino acid peptide is released from the heart and circulates in the body in both rats and humans ( Biochem.Biophys.Res.Comm. 129 (1985), 439 - 446; and Science 229 (1985), 397 - 400). The primaty structure of the 28 amino acid peptide from rat is

```
H-Ser-Leu-Arg-Arg-Ser-Ser-Cys-Phe-Gly-Gly-Arg-Ile-Asp-Arg
                    5     |          10                 |
                          S                             |
                          |                        15 Ile
                          S                             |
                          |                             |
  HO-Tyr-Arg-Phe-Ser-Asn-Cys-Gly-Leu-Gly-Ser-Gln-Ala-Gly
                    25               20
```

The sequence of the corresponding human peptide differs by only one amino acid, i.e. Met in position 12.

Hereinafter, the rat atrial natriuretic and vasoactive peptide will be referred to by the abbreviation ANP and subscripts will be used to indicate specific sequences with ANP-(1-28) (numbered from amino to carboxy terminus) serving as the reference polypeptide. Hence, ANP-(5-28) is ANP from which H-$Ser^1$-$Leu^2$-$Arg^3$-$Arg^4$-is omitted. [N-$MeSer^{19}$]-ANP is ANP wherein one hydrogen atom in the $\alpha$-amino group in $Ser^{19}$ is substituted by a methyl group (hereinafter designated Me). [$Leu^{18}$]-ANP is ANP wherein $Gln^{18}$ is substituted by Leu. ANP-$Pro^{29}$-$NH_2$ is ANP elongated at the C-terminus with Pro-$NH_2$. The abbreviation hANP is the human analog to ANP, i.e., $Ile^{12}$ is substituted by Met. Unless otherwise indicated, the amino acids are in L-configuration.

Several derivatives of ANP and hANP are known. However, no ANP or hANP derivatives in which two amino acid residues in the 7-23 ring system are exchanged with other amino acid residues compared with ANP and hANP are known. Furthermore, no ANP or hANP derivatives which in the C terminal end are elongated with Pro, Trp, Phe or N-MePhe, are known.

In accordance with this invention, novel atrial natriuretic/vasodilator polypeptide compounds (ANP) are provided for the regulation of fluid volume and blood pressure in host organisms, in which one aspect of the invention provides ANPs substantially free of unrelated atrial tissue or products.

Generally, the present invention relates to novel compounds of the general formula I

$R^a$-$Ser_{n1}$-$Leu_{n2}$-$Arg_{n3}$-$(R^4)_{n4}$-$(R^5)_{n5}$-$(R^6)_{n6}$-Cys-$R^8$-$R^9$-$R^{10}$-$R^{11}$-
$R^{12}$-$R^{13}$-$R^{14}$-Ile-$R^{16}$-$R^{17}$-$R^{18}$-$R^{19}$-$R^{20}$-$R^{21}$-$R^{22}$-Cys-$R^{24}$-$R^{25}$-$R^{26}$-
$R^{27}$-$R^{28}$-$(R^{29})_{n29}$-$(R^{30})_{n30}$-$(R^{31})_{n31}$-$(R^{32})_{n32}$-$(R^{33})_{n33}$-$(R^{34})_{n34}$-
$(R^{35})_{n35}$-$(R^{36})_{n36}$-$(R^{37})_{n37}$ $(R^{38})_{n38}$-$R^b$ (I)

wherein $R^4$ is Arg, Lys or D-Arg, $R^5$ is Ser or Ile, $R^6$ is Ser, D-Ser or Asp, $R^8$ is Phe, Leu, N-Bzl-Gly, Orn(2-pyrimidinyl) or Trp, $R^9$ is Gly, D-Trp, D-Ala or D-Phe, $R^{10}$ is Gly, Asp or Pro, $R^{11}$ is Arg, Lys, Gln or Orn(2-pyrimidinyl), $R^{12}$ is Ile, Met or Trp, $R^{13}$ is Asp, Glu, Asn, Lys or Arg, $R^{14}$ is Arg, Lys, Orn(2-pyrimidinyl) or Asp, $R^{16}$ is Gly, D-Leu, D-Ala or D-Arg, preferably Gly, $R^{17}$ is Ala, Leu, Ile, Lys or Arg, $R^{18}$ is Gln, Arg, Lys, Leu, D-Leu or Ile, $R^{19}$ is Ser, Ile, Ala or Asp, $R^{20}$ is Gly, D-Ala, Val, D-Phe, Lys or Arg, $R^{21}$ is Leu, Ile or Trp, $R^{22}$ is Gly, D-Ala, Sar, D-Phe or Ala, $R^{24}$ is Asn, Pro, N-MeArg or N-MeAsn, $R^{25}$ is Ser, D-Ser or Ala, $R^{26}$ is Phe, D-Phe, N-MePhe, N-Bzl-Gly or Ile, $R^{27}$ is Arg, Lys, D-Arg, Orn(2-pyrimidinyl) or N-MeArg, $R^{28}$ is Tyr, D-

Tyr, Pro or Trp, $R^{29}$ is Pro, Trp, Phe or N-MePhe, $R^{30}$ is Gln, Arg, Lys, Glu, Asp-$\beta$-(NH-phenethyl), Ala, Asn or Phe, $R^{31}$ is Tyr, Pro, Ser or Trp, $R^{32}$ is Phe, Leu or Tyr, $R^{33}$ is Gln, Glu, Leu or Arg, $R^{34}$ is Ser, Glu or Phe, $R^{35}$ is Phe, Ser or Leu, $R^{36}$ is Leu or Phe, $R^{37}$ is Arg or Gln, $R^{38}$ is Ser or Arg, or $R^8$ together with $R^9$ represents a group of the formula $-NH-m-C_6H_4-CH_2CO-$, $R^9$ together with $R^{10}$ represents a group of the formula $-NH-CH_2-m-C_6H_4-CO-$, $R^{21}$ together with $R^{22}$ represents a group of the formula $-NH-CH_2-m-C_6H_4-CO-$, n1, n2, n3, n4, n5, n6, n29, n30, n31, n32, n33, n34, n35, n36, n37 and n38 are the same or different and each represent zero or one, $R^a$ is hydrogen or acyl, and $R^b$ is hydroxy, amino or substituted amino such as alkylamino, N,N-dialkylamino, adamantylamino or aralkylamino such as phenethylamino, and wherein there is a disulfide bridge between $Cys^7$ and $Cys^{23}$, with the proviso that either at least two of the amino acid residues $R^8$, $R^9$, $R^{10}$, $R^{11}$, $R^{12}$, $R^{13}$, $R^{14}$, $R^{16}$, $R^{17}$, $R^{18}$, $R^{19}$, $R^{20}$, $R^{21}$ and $R^{22}$ are different from Phe, Gly, Gly, Arg, Ile or Met, Asp, Arg, Gly, Ala, Gln, Ser, Gly, Leu and Gly, respectively, or if the amino acid residue $R^{12}$ is Ile or Met, and the amino acid residues $R^8$, $R^9$, $R^{10}$, $R^{11}$, $R^{13}$, $R^{14}$, $R^{16}$, $R^{17}$, $R^{18}$, $R^{19}$, $R^{20}$, $R^{21}$ and $R^{22}$ are Phe, Gly, Gly, Arg, Asp, Arg, Gly, Ala, Gln, Ser, Gly, Leu and Gly, respectively, then n29 is one, and salts thereof.

Herein alkyl preferably is a lower alkyl group, preferably containing less than 8 carbon atoms, more preferred less than 5 carbon atoms. Analogously, this applies for groups containing an alkyl group, such as alkoxy, alkanoyl, N,N-dialkylamino.

The preferred compounds of formula I can be prepared on the basis of the examples given below, and on the basis of methods which are generally known in peptide syntheses. Briefly, compounds of formula I can be "built up" entirely by conventional peptide synthesis in solution, entirely by solid phase peptide synthesis or by a combination of conventional and solid phase peptide synthesis.

Common to the synthesis of the preferred compounds of formula I is the protection of the labile side chain groups, which will prevent an undesired chemical reaction from occurring at these sites until the groups are ultimately removed. Such a protection is designated a permanent protection. Also common is the protection of an $\alpha$-amino group in an amino acid or a peptide fragment while that entity reacts at the carboxyl group, followed by the selective removal of the $\alpha$-amino protecting group to allow subsequent reaction to take place at that location. Such a protection is designated a temporary protection. Accordingly, it is common that as a step in the synthesis an intermediate peptide fragment is produced which includes each of the amino acid residues located in its desired position in the peptide chain with various of these residues linked to the side chain permanent protecting groups and with the carboxylic acid in the C-terminal protected with a group that can be removed selectively without interfering with the permanent protection, but at the same time being stable during the conditions used to remove the $\alpha$-amino protecting group. Such a protection is designated a semipermanent protection.

The conventional peptide synthesis in solution is performed essentially at known technique as described by M. Bodanszky in Principles of Peptide Synthesis (Springer-Verlag 1984). Among the classes of temporary protection groups mentioned are for the synthesis here selected benzyloxycarbonyl (hereinafter designated Z) and fluorenylmethyloxycarbonyl (hereinafter designated Fmoc) and among the classes of permanent protection groups, t-butyloxycarbonyl (hereinafter designated Boc), t-butyl (hereinafter designated $Bu^t$), S-acetamidomethyl (hereinafter designated Acm) and protonation of the guanidino groups. Among the classes of semipermanent protection groups, methoxy (hereinafter designated OMe) is selected.

Compounds of the general formula I can be built up by methods of conventional peptide synthesis in solution in analogy to procedures described by M. Bodanszky in Principles of Peptide Synthesis (Springer-Verlag 1984) and in The Peptides 1-5, Ed. E. Gross and J. Meienhofer (Academic Press 1979) using conveniently protected ANP-fragments. Mainly, the protected fragments are prepared by the mixed anhydride method using isobutylchloroformate (hereinafter designated IOC-Cl), and the protected fragments are condensated by the mixed anhydride method or by the N-(N,N-dimethylaminopropyl)-N'-ethylcarbodiimide/1-hydroxybenzotriazole method (hereinafter designated EDAC/HOBt). The fully protected peptide so obtained can be deprotected under mildly acid conditions, e.g. by treatment with trifluoroacetic acid (hereinafter designated TFA) to obtain the peptide in its linear form still containing the Acm group on all cysteines. The cyclic form of the peptide is obtained by oxidation using iodine in 80% acetic acid. The crude peptide is subjected to semipreparative high pressure liquid chromatography purification (hereinafter designated HPLC) as described in Example 1 below.

The peptides in this application are preferably prepared by solid phase peptide synthesis analogous to procedures described by J.M. Stewart and J.D. Young in Solid Phase Peptide Synthesis, 2nd edition (1984), using the methodology described in Example 1 below. Among the classes of temporary protecting groups for this procedure are selected Boc and among the classes of permanent protection groups, benzyl (hereinafter designated Bzl), 4-bromobenzyloxycarbonyl (hereinafter designated Br-Z), 4-methoxybenzyl (hereinafter designated Mob), 4-toluenesulfonyl (hereinafter designated Tos) and mesitylene-2-sulfonyl

(hereinafter designated Mts). The resin utilized is of the so-called "Pam" polystyrene type as described in J.Org.Chem . 43 (1978), 2845 - 52, or the so-called "MBHA" polystyrene type as described in Peptides 2 (1981), 45 -50.

The synthesis is performed essentially as described in Example 1, using an Applied Biosystems 430 A automatically peptide synthesizer. The amino acids are preferably activated as symmetrical anhydrides or as 1-hydroxybenzotriazole (hereinafter designated HOBt) esters. Each protected amino acid is introduced into the solid phase reactor in about a two to fourfold excess, and the coupling is mainly carried out in dimethylformamide (hereinafter designated DMF).

After the desired amino acid sequence has been completed, the intermediate peptide is removed from the resin support by treatment with a reagent, such as liquid hydrogen fluoride either by the low-high procedure as described in J.Amer.Chem.Soc. 105 (1983), 6442 - 55, or by HF/anisole/methylethylsulfide/90:5:5 as described in Biochem.Biophys.Res.Comm. 131 (1985), 1056 - 62. These procedures removes at the same time all side chain protecting groups leaving the peptide in its linear form. The cyclic form of the peptide is obtained by oxidizing the linear form in a ferrycyanide solution, preferably as described in Biochem.Biophys.Res.Comm. 131 No. 3 (1985), 1056 - 62, or in accordance with other known procedures. The crude peptide is subjected to semipreparative HPLC purification as described in Example 1 below.

Alternatively, some compounds of this invention can be produced by expression of recombinant DNA constructs. Such production can be desirable to provide large quantities or alternative embodiments of such compounds.

More particularly, modifications in the amino acid sequence of the various forms of pre-proANP, proANP and ANP compounds can be effected by various changes in the nucleotide sequence of the cloned structural gene used to direct the synthesis of compounds of formula I. Included within such modification of the DNA sequence are the replacement of various codons with other codons which, due to the degeneracy of the genetic code, direct the synthesis of the same amino acid.

In addition, by codon substitution, one or more amino acid residues can be replaced by functionally equivalent residues.

Specific examples of preferred and interesting compounds of formula I are as follows:

| Internal Code NO-70- | Compound |
|---|---|
| 0237 | ANP-(5-28)-$Pro^{29}$-$NMe_2$ |
| 0270 | [$N^{\alpha}$-acetyl-$Arg^4$,D-$Ala^9$,$Leu^{18}$,D-$Ala^{20}$,D-$Ala^{22}$,-N-$MePhe^{26}$,D-$Tyr^{28}$]ANP-(4-28)-$Pro^{29}$-$NH_2$ |
| 0273 | ANP-(5-28)-N-$MePhe^{29}$-$NH_2$ |
| 0317 | [N-(4-guanidinobenzoyl)-$Ser^5$]ANP-(5-28)-N-$MePhe^{29}$-$NH_2$ |
| 0337 | [N-(4-guanidinobutyryl)-$Ser^5$]ANP-(5-28)-N-$MePhe^{29}$-$NH_2$ |
| 0338 | [$N^{\alpha}$-acetyl-D-$Arg^4$,D-$Ala^9$,$Gln^{11}$,$Leu^{18}$,D-$Ala^{20}$,D-$Ala^{22}$,N-$MePhe^{26}$,$Pro^{28}$]ANP-(4-28)-$Pro^{29}$-$NH_2$ |
| 0355 | [$N^{\alpha}$-acetyl-D-$Arg^4$,D-$Ala^9$,$Gln^{11}$,D-$Ala^{16}$,D-$Leu^{18}$,-D-$Ala^{20}$,D-$Ala^{22}$,$Ala^{25}$,N-$MePhe^{26}$,D-$Tyr^{28}$]ANP-(4-28)-$Pro^{29}$-$NH_2$ |
| 0351 | [$Arg^{17}$,$Ile^{18}$]ANP-(5-28) |
| 0352 | [$N^{\alpha}$-acetyl-$Arg^4$,D-$Ala^9$,$Leu^{18}$,D-$Ala^{20}$,D-$Ala^{22}$,-N-$MePhe^{26}$,D-$Tyr^{28}$]ANP-(4-28)-$Pro^{29}$-Asp-β(NH-phenethyl)$^{30}$-$NH_2$ |
| 0354 | [$N^{\alpha}$-acetyl-$Arg^4$,Orn(2-pyrimidinyl)$^8$,D-$Ala^9$,$Leu^{18}$,-D-$Ala^{20}$,D-$Ala^{22}$,N-$MePhe^{26}$,D-$Tyr^{28}$]ANP-(4-28)-$Pro^{29}$-$NH_2$ |
| 0353 | [$N^{\alpha}$-acetyl-$Arg^4$,D-$Ala^9$,$Leu^{18}$,D-$Ala^{20}$,m-(NH-methyl)-benzoyl$^{21-22}$,N-$MePhe^{26}$,D-$Tyr^{28}$]ANP-(4-28)-$Pro^{29}$-$NH_2$ |
| 0362 | [$N^{\alpha}$-acetyl-$Arg^4$,D-$Ala^9$,$Leu^{18}$,D-$Ala^{20}$,D-$Ala^{22}$,N-$MePhe^{26}$,$Pro^{28}$]ANP-(4-28)-$Pro^{29}$-$NH_2$ |
| 0371 | ANP-(5-28)-$Pro^{29}$-NH-(1-adamantyl) |

0372 ANP-(5-28)-Pro$^{29}$-NH-lauryl

0425 [N$^{\alpha}$-acetyl-Arg$^{4}$,D-Ala$^{9}$,Met$^{12}$,Leu$^{18}$,D-Ala$^{20}$,D-Ala$^{22}$,N-MePhe$^{26}$,D-Tyr$^{28}$]ANP-(4-28)-Pro$^{29}$-NH$_{2}$

0377 [N$^{\alpha}$-acetyl-Arg$^{4}$,D-Ala$^{9}$,Leu$^{18}$,D-Ala$^{20}$,D-Ala$^{22}$,N-MePhe$^{26}$]ANP-(4-28)-Phe$^{29}$-Gln$^{30}$-Tyr$^{31}$-Phe$^{32}$-NH$_{2}$

0378 [N$^{\alpha}$-acetyl-Arg$^{4}$,D-Ala$^{9}$,Leu$^{18}$,D-Ala$^{20}$,D-Ala$^{22}$,N-MePhe$^{26}$,D-Tyr$^{28}$]ANP-(4-28)-Pro$^{29}$-Arg$^{30}$-Pro$^{31}$-NH$_{2}$

0395 [N$^{\alpha}$-acetyl-Arg$^{4}$,D-Ala$^{9}$,Leu$^{18}$,D-Ala$^{20}$,D-Ala$^{22}$,N-MePhe$^{26}$,D-Tyr$^{28}$]ANP-(4-28)-Pro$^{29}$-Glu$^{30}$-NH$_{2}$

0392 [N$^{\alpha}$-acetyl-Arg$^{4}$,D-Ala$^{9}$,Leu$^{18}$,D-Phe$^{20}$,D-Ala$^{22}$,N-MePhe$^{26}$,D-Tyr$^{28}$]ANP-(4-28)-Pro$^{29}$-NH$_{2}$

0400 [N$^{\alpha}$-acetyl-Arg$^{4}$,D-Ala$^{9}$,Trp$^{12}$,Leu$^{18}$,D-Ala$^{20}$,D-Ala$^{22}$,N-MePhe$^{26}$,D-Tyr$^{28}$]ANP-(4-28)-Pro$^{29}$-NH$_{2}$

0410 [N$^{\alpha}$-acetyl-Arg$^{4}$,m-NH-phenylacetyl$^{8-9}$,Leu$^{18}$,D-Ala$^{20}$,D-Ala$^{22}$,N-MePhe$^{26}$,D-Tyr$^{28}$]ANP-(4-28)-Pro$^{29}$-NH$_{2}$

0422 [N$^{\alpha}$-acetyl-Arg$^{4}$,D-Ala$^{9}$,Asp$^{10}$,Leu$^{18}$,D-Ala$^{20}$,D-Ala$^{22}$,N-MePhe$^{26}$,D-Tyr$^{28}$]ANP-(4-28)-Pro$^{29}$-NH$_{2}$

0401 [N$^{\alpha}$-acetyl-Arg$^{4}$,m-(NH-methyl)benzoyl$^{9-10}$,Leu$^{18}$,D-Ala$^{20}$,D-Ala$^{22}$,N-MePhe$^{26}$,D-Tyr$^{28}$]ANP-(4-28)-Pro$^{29}$-NH$_{2}$

0397 [N$^{\alpha}$-acetyl-Arg$^{4}$,Trp$^{8}$,D-Ala$^{9}$,Leu$^{18}$,D-Ala$^{20}$,D-Ala$^{22}$,N-MePhe$^{26}$,D-Tyr$^{28}$]ANP-(4-28)-Pro$^{29}$-NH$_{2}$

0389 [N$^{\alpha}$-acetyl-Arg$^{4}$,D-Ala$^{9}$,Leu$^{18}$,D-Ala$^{20}$,D-Ala$^{22}$,N-MePhe$^{26}$,Trp$^{28}$]ANP-(4-28)-Trp$^{29}$-NH$_{2}$

0390 [N$^{\alpha}$-acetyl-Arg$^{4}$,D-Trp$^{9}$,Leu$^{18}$,D-Ala$^{20}$,D-Ala$^{22}$,N-MePhe$^{26}$,D-Tyr$^{28}$]ANP-(4-28)-Pro$^{29}$-NH$_{2}$

0391 [N$^{\alpha}$-acetyl-Arg$^{4}$,D-Ala$^{9}$,D-Leu$^{16}$,Leu$^{18}$,D-Ala$^{20}$,D-Ala$^{22}$,N-MePhe$^{26}$,D-Tyr$^{28}$]ANP-(4-28)-Pro$^{29}$-NH$_{2}$

| | |
|---|---|
| 0405 | [N$^{\alpha}$-acetyl-Arg$^{4}$,D-Ala$^{9}$,Leu$^{18}$,D-Ala$^{20}$,D-Ala$^{22}$,N-MePhe$^{26}$,D-Tyr$^{28}$]ANP-(4-28)-Pro$^{29}$-Gln$^{30}$-NH$_{2}$ |
| 0404 | [N$^{\alpha}$-acetyl-Arg$^{4}$,D-Ala$^{9}$,Leu$^{18}$,D-Ala$^{20}$,D-Ala$^{22}$,N-MePhe$^{26}$]ANP-(4-28)-Pro$^{29}$-NH$_{2}$ |
| 0403 | [N$^{\alpha}$-acetyl-Arg$^{4}$,D-Ala$^{9}$,Leu$^{18}$,D-Ala$^{20}$,Trp$^{21}$,D-Ala$^{22}$,N-MePhe$^{26}$,D-Tyr$^{28}$]ANP-(4-28)-Pro$^{29}$-NH$_{2}$ |
| 0420 | [N$^{\alpha}$-acetyl-Arg$^{4}$,D-Ala$^{9}$,Gln$^{11}$,Arg$^{13}$,Asp$^{14}$,Leu$^{18}$,D-Ala$^{20}$,D-Ala$^{22}$,N-MePhe$^{26}$,D-Tyr$^{28}$]ANP-(4-28)-Pro$^{29}$-NH$_{2}$ |
| 0406 | [N$^{\alpha}$-acetyl-Arg$^{4}$,D-Ala$^{9}$,Leu$^{18}$,D-Ala$^{20}$,D-Ala$^{22}$,N-MePhe$^{26}$,N-MeArg$^{27}$,D-Tyr$^{28}$]ANP-(4-28)-Pro$^{29}$-NH$_{2}$ |
| 0431 | [N$^{\alpha}$-acetyl-Arg$^{4}$,D-Phe$^{9}$,D-Ala$^{16}$,Leu$^{18}$,D-Ala$^{20}$,D-Ala$^{22}$,N-MePhe$^{26}$,Pro$^{28}$]ANP-(4-28)-Pro$^{29}$-NH$_{2}$ |
| 0432 | [N$^{\alpha}$-acetyl-Arg$^{4}$]ANP-(4-28)-Phe$^{29}$-Gln$^{30}$-Tyr$^{31}$-Phe$^{32}$-NH$_{2}$ |
| 0433 | [N$^{\alpha}$-acetyl-Arg$^{4}$,Pro$^{28}$]ANP-(4-28)-Pro$^{29}$-NH$_{2}$ |
| 0542 | [N$^{\alpha}$-acetyl-Arg$^{4}$,D-Ala$^{9}$,Leu$^{18}$,D-Ala$^{22}$,N-MePhe$^{26}$,Pro$^{28}$]ANP-(4-28)-Pro$^{29}$-NH$_{2}$ |
| 0597 | [N$^{\alpha}$-acetyl-Arg$^{4}$,N-MePhe$^{26}$,Pro$^{28}$]ANP-(4-28)-Pro$^{29}$-NH$_{2}$ |
| 0651 | [N$^{\alpha}$-acetyl-Arg$^{4}$,D-Ala$^{9}$,D-Arg$^{16}$,Leu$^{18}$,D-Ala$^{20}$,D-Ala$^{22}$,N-MePhe$^{26}$,D-Tyr$^{28}$]ANP-(4-28)-Pro$^{29}$-NH$_{2}$ |
| 0600 | [N$^{\alpha}$-acetyl-Arg$^{4}$,D-Tyr$^{28}$]ANP-(4-28)-Pro$^{29}$-NH$_{2}$ |
| 0662 | [N$^{\alpha}$-acetyl-Arg$^{4}$,D-Ala$^{9}$,Pro$^{10}$,Leu$^{18}$,D-Ala$^{20}$,D-Ala$^{22}$,N-MePhe$^{26}$,D-Tyr$^{28}$]ANP-(4-28)-Pro$^{29}$-NH$_{2}$ |
| A | [N$^{\alpha}$-acetyl-Arg$^{4}$,D-Ala$^{9}$,Leu$^{18}$,D-Ala$^{20}$,D-Ala$^{22}$,N-MePhe$^{26}$]ANP-(4-28)-Phe$^{29}$-Gln$^{30}$-Tyr$^{31}$-Phe$^{32}$-Gln$^{33}$-Ser$^{34}$-Phe$^{35}$-NH$_{2}$ |

0654 [$N^{\alpha}$-acetyl-$Arg^{4}$,D-$Ala^{9}$,$Leu^{18}$,D-$Ala^{20}$,D-$Ala^{22}$,N-$MePhe^{26}$]ANP-(4-28)-$Phe^{29}$-$Arg^{30}$-$Tyr^{31}$-$Phe^{32}$-$Glu^{33}$-$NH_2$

B [$N^{\alpha}$-acetyl-$Arg^{4}$,D-$Ala^{9}$,$Leu^{18}$,D-$Ala^{20}$,D-$Ala^{22}$,N-$MePhe^{26}$]ANP-(4-28)-$Phe^{29}$-$Gln^{30}$-$Ser^{31}$-$Leu^{32}$-$Leu^{33}$-$Glu^{34}$-$Ser^{35}$-$Leu^{36}$-$Arg^{37}$-$NH_2$

C [$N^{\alpha}$-acetyl-$Arg^{4}$,D-$Ala^{9}$,$Leu^{18}$,D-$Ala^{20}$,D-$Ala^{22}$,N-$MePhe^{26}$]ANP-(4-28)-$Gln^{29}$-$Phe^{30}$-$Ser^{31}$-$Leu^{32}$-$Arg^{33}$-$Phe^{34}$-$NH_2$

D [$N^{\alpha}$-acetyl-$Arg^{4}$,D-$Ala^{9}$,$Leu^{18}$,D-$Ala^{20}$,D-$Ala^{22}$,N-$MePhe^{26}$]ANP-(4-28)-$Pro^{29}$-$Ala^{30}$-$Trp^{31}$-$Tyr^{32}$-$NH_2$

E [$N^{\alpha}$-acetyl-$Arg^{4}$,D-$Ala^{9}$,$Leu^{18}$,D-$Ala^{20}$,D-$Ala^{22}$]ANP-(4-28)-$Phe^{29}$-$Gln^{30}$-$Ser^{31}$-$Leu^{32}$-$Leu^{33}$-$Ser^{34}$-$Leu^{35}$-$Leu^{36}$-$Gln^{37}$-$Ser^{38}$-$NH_2$

and salts thereof, e.g. hydrochlorides, sulphates or acetates.

Phenethyl is preferably β-phenethyl.

In one preferred embodiment of this invention, $R^a$ is different from hydrogen.

In a further preferred embodiment of this invention, $R^a$ is acyl, preferably alkanoyl, aminoalkylcarbonyl, aralkoxycarbonyl or 4-guanidinobenzoyl or 4-guanidinobutyryl.

In a further preferred embodiment of this invention, $R^a$ is acetyl.

In a further preferred embodiment of this invention, $R^b$ is different from hydroxy

In a further preferred embodiment of this invention, $R^b$ is amino or N,N-dialkylamino, preferably amino.

In a further preferred embodiment of this invention, $R^4$ is Arg or D-Arg, preferably Arg.

In a further preferred embodiment of this invention, $R^5$ is Ser.

In a further preferred embodiment of this invention, $R^6$ is Ser.

In a further preferred embodiment of this invention, $R^8$ is Phe or Trp, preferably Phe.

In a further preferred embodiment of this invention, $R^9$ is D-Trp, D-Ala or D-Phe, preferably D-Ala.

In a further preferred embodiment of this invention, $R^{10}$ is Gly.

In a further preferred embodiment of this invention, $R^{11}$ is Arg or Gln, preferably Arg.

In a further preferred embodiment of this invention, $R^{12}$ is Ile or Met, preferably Ile.

In a further preferred embodiment of this invention, $R^{13}$ is Asp or Glu, preferably Asp.

In a further preferred embodiment of this invention, $R^{14}$ is Arg.

In a further preferred embodiment of this invention, $R^{17}$ is Ala or Arg, preferably Ala.

In a further preferred embodiment of this invention, $R^{18}$ is Gln, Leu or Ile, preferably Leu.

In a further preferred embodiment of this invention, $R^{19}$ is Ser.

In a further preferred embodiment of this invention, $R^{20}$ is Gly, D-Ala or D-Phe, preferably D-Ala.

In a further preferred embodiment of this invention, $R^{21}$ is Leu.

In a further preferred embodiment of this invention, $R^{22}$ is Gly or D-Ala, preferably D-Ala.

In a further preferred embodiment of this invention, $R^{24}$ is Asn.

In a further preferred embodiment of this invention, $R^{25}$ is Ser.

In a further preferred embodiment of this invention, $R^{26}$ is Phe, N-MePhe or N-Bzl-Gly, preferably N-MePhe.

In a further preferred embodiment of this invention, $R^{27}$ is Arg or N-MeArg, preferably Arg.

In a further preferred embodiment of this invention, $R^{28}$ is Tyr, D-Tyr or Pro, preferably D-Tyr or Pro.

In a further preferred embodiment of this invention, $R^{29}$ is Pro, Phe, or N-MePhe, preferably Pro or Phe,

most preferred Pro.

In a further preferred embodiment of this invention, $R^{30}$ is Asp-$\beta$-(NH-phenethyl), Gln or Arg.

In a further preferred embodiment of this invention, $R^{31}$ is Tyr or Pro.

In a further preferred embodiment of this invention, $R^{32}$ is Phe.

In a further preferred embodiment of this invention, n1, n2 and n3 each is zero.

In a further preferred embodiment of this invention, n29 is one.

In a further preferred embodiment of this invention, n30 is one.

In a further preferred embodiment of this invention, n31 is one.

In a further preferred embodiment of this invention, n32 is one.

In a further preferred embodiment of this invention, n33, n34, n35, n36, n37 and n38 each is zero.

Compounds of this invention have natriuretic and diuretic activity in the intact mammal and in the kidney isolated from a mammal. Furthermore, compounds of this invention possess vasorelaxant activity and inhibit the release of aldosterone.

Some compounds of formula I have advantageous prolonged activities.

Compounds of this invention which are shown to have the above recited physiological effects can find use in numerous therapeutical applications such as, for example, inducing natriuresis, diuresis and vasodilation. Thus these compounds can find use as therapeutic agents in the treatment of various oedematous states such as, for example, congestive heart failure, nephrotic syndrome and hepatic cirrhosis, in addition to hypertension and renal failure due to ineffective renal perfusion or reduced glomerular filtration rate.

Compounds of formula I can be administered to mammals for veterinary use such as with domestic animals, and clinical use in humans in a manner similar to other therapeutic agents, that is in a physiologically acceptable carrier. In general the dosage of compounds of formula I will range from about 0.1 to 100 µg/kg, more usually from about 0.1 to 10 µg/kg of the host body weight. Alternatively, dosages within these ranges can be administered by constant infusion over an extended period of time, usually exceeding 24 hours, until the desired therapeutic benefits have been obtained.

Compounds of formula I can be administered neat, as mixtures with other physiologically acceptable active or inactive materials, or with physiologically suitable carriers such as, for example, water or normal saline. Compounds of formula I can be administered nasally or parenterally, for example, by injection. The administration of compounds of formula I can be performed by subcutaneous, intravenous or intramuscular injection.

Compounds of formula I are desirably administered in pharmaceutically effective amounts and often as pharmacologically acceptable salts such as acid addition salts. Such salts can include, for example, hydrochloride, hydrobromide, phosphate, sulphate, acetate, benzoate, maleate, among others.

Compounds of this invention can also be used for preparing antisera for use in immunoassays employing labelled reagents, usually antibodies.

Abbreviations used are in accordance with the rules approved (1974) by the IUPAC-IUB Commission on Biochemical Nomenclature, vide collected Tentative Rules & Recommendations of the commission on biochemical Nomenclature IUPAC-IUB, 2nd ed., Maryland 1975. Herein, m-$C_6H_4$ designates metha phenylene.

## BIOLOGICAL TESTS

### Test for diuretic activity

Female rats weighing about 200 to 250 g were anaestetized intraperitoneally with pentobarbital sodium (50 mg/kg body weight). Catheters (Clay-Adams PE 50) were implanted in both jugular veins for injection and infusion and in one carotid artery for blood pressure registration. The carotid catheter was connected to a pressure transducer filled with heparin-saline. Through a laparotomy catheters (Clay-Adams PE 10) were implanted in both ureters. The urine flowing from the catheters was collected in a glass suspended in an isometric force transducer giving a continuous registration of the weight of the excreted urine. The rat was given a saline load of 8 to 10 ml infused over a period of 30 minutes. The test substance was given as a bolus injection. Each rat received only one dose of only one compound. Two or three doses of each compound were tested and the dose-response curve was compared with a similar dose-response curve obtained with ANP-(5-28).

Test for vasodilating activity (in vitro)

A rabbit (NZ white) was killed by a blow to the neck and exsanguination. The left renal artery was removed, cut helically and immediately suspended in an organ bath containing Krebs' solution kept at 37°C and aerated with 5% carbon dioxide in 95% oxygen. Contractions of the preparation were recorded isotonically, the tension being 860 mg.

After the preparation had been mounted in the organ bath, a relaxation occured. When this relaxation had faded, doses of noradrenaline were added to the bath until the response was stable. During this period of time, a maximal (100%) contraction should be achieved at least once. Before each dose of noradrenaline, a period of full relaxation lasting for at least 5 minutes was allowed to pass.

In order to standardize the test so that the activity of different atrial natriuretic peptides can be characterized and compared in a uniform way, a concentration of noradrenaline was found which leads to a contraction of 30 to 40% of the maximal contraction. Against this fixed noradrenaline concentration, the peptides were tested in two or three concentrations and the concentration-response curves were compared with a similar curve obtained with ANP-(5-28).

Effect on blood pressure in rats

Female rats weighing about 200 g were anaesthetized intraperitoneally with pentobarbital sodium (62 mg/kg body weight). One PE-50 catheter was placed in the jugular vein and used for intravenous infusion. Another catheter was placed in the carotid artery and connected to a Gould pressure transducer and a Kip and Zonen recorder for registration of arterial blood pressure. Finally, a catheter was introduced in the bladder via a small midline incision. Urine was collected in small vials and diuresis determined by weight. However, due to the collection via the bladder, only a rough estimation of diuresis was obtained.

When surgery had been completed, the rat was placed under a lamp and an intravenous infusion of human serum albumin (0.1% in 0.9% saline) was started at an infusion rate of 3.8 ml/h. When blood pressure had stabilized, the atrial peptide was infused intravenously (3.8 ml/h). After 20 minutes, the infusion was stopped but blood pressure was still measured during the following 30 minutes. The effect was determined as the fall in blood pressure compared to the control period. The duration of effects of about 20 mm Hg was estimated after the termination of the infusion at the time when blood pressure rose to a level corresponding to only two thirds of the total fall in blood pressure.

Only one dose of one atrial peptide was tested in each experiment. The relative potency compared with ANP-(5-28) was determined by comparing the dose-response curves.

| Compound | Internal code NO-70- | relative vaso-dilating activity in vitro | Relative diuretic activity in rats | Relative effect on blood pressure in rats |
|---|---|---|---|---|
| ANP-(5-28) | | 1 | 1 | 1 |
| [N-(p-guanidinobenzoyl)-$Ser^{5}$]-ANP-(5-28)-N-$MePhe^{29}$-$NH_2$ | 0317 | 2 | 1 | 1.5 |
| ANP-(5-28)-N-$MePhe^{29}$-$NH_2$ | 0273 | 5 | 2 | 1.3 |
| ANP-(5-28)-$Pro^{29}$-$NMe_2$ | 0237 | 4 | 1.5 | 1.3 |
| [N-(4-guanidinobutyryl)-$Ser^{5}$]-ANP-(5-28)-N-$MePhe^{29}$-$NH_2$ | 0337 | 3 | 1 | 0.8 |
| ANP-(5-28)-$Pro^{29}$-NH-(1-adamantyl) | 0371 | 1 | 1 | 2 |
| [$N^{\alpha}$-acetyl-$Arg^{4}$]ANP-(4-28)-$Phe^{29}$-$Gln^{30}$-$Tyr^{31}$-$Phe^{32}$-$NH_2$ | 0432 | 1 | 0.7 | |

Example 1

ANP-(5-28)-N-MePhe$^{29}$-$NH_2$ was prepared entirely by solid phase peptide synthesis.

Assembly of the protected ANP-(5-28)-N-MePhe$^{29}$-$NH_2$ sequence on the resin was performed as described below. The resin utilized was of the so-called "MBHA" type which is described in G.R. Matsueda and J.M. Stewart: Peptides 2 (1981), 45 - 50 obtained from Applied Biosystems, United States (part No. 400229).

The following commercially available amino acid derivatives were applied: Boc-Arg(Tos)-OH, Boc-Phe-OH, Boc-Asp(obzl)-OH, Boc-Ser(Bzl)-OH, Boc-Asn-OH, Boc-Cys(Mob)-OH, BOC-GLY-OH, BOC-Leu-OH, Boc-Gln-OH, Boc-Ile-OH, Boc-Ala-OH, Boc-Tyr(Br-Z)-OH and Boc-N-MePhe-OH.

The assembly of the peptide chain was done successively by an automatic peptide synthesizer (Applied Biosystems United States, model 430A).

The incorporation of one amino acid consisted essentially of the following operations:

1) Removal of the Boc-protecting group from the previously attached amino acid by TFA/$CH_2Cl_2$ - (70:30) for 20 minutes at room temperature followed by washing with $CH_2Cl_2$.

2) Convertion of the protonated amino group to its unprotonated form by treatment with diisopropylethylamine/DMF (1:6) followed by washing with DMF.

3) Coupling with the appropriately protected amino acid derivative either as the symmetrical anhydride (2 equivalents) or as the HOBt ester (4 equivalents) to the resin bound peptide for 16 - 40 minutes at room temperature followed by a two step washing procedure with DMF and with $CH_2Cl_2$, respectively.

All the amino acid derivatives were coupled as symmetrical anhydrides except for Boc-Asn-OH, Boc-Gln-OH and Boc-Arg(Tos)-OH which were coupled as their HOBt esters. In order to improve the yield of the couplings they were repeated in the cases of Boc-Asn-OH, Boc-Gln-OH, Boc-Arg(Tos)-OH, Boc-Tyr(Br-Z)-OH and Boc-N-MePhe-OH.

The HOBt esters were automatically prepared by a reaction of the amino acid derivatives HOBt (1 equivalent) and dicyclohexyl carbodiimide (hereinafter designated DCC) (1 equivalent) in DMF for 25 minutes at room temperature. The resulting solution was filtered and used directly for coupling.

The symmetrical anhydrides were automatically prepared by a reaction of the amino acid derivative with DCC (0.5 equivalents) in $CH_2Cl_2$ for 6 minutes at room temperature. After filtration, the $CH_2Cl_2$ was evaporated and replaced by DMF and the resulting solution was used for coupling.

After coupling of Ala$^{17}$ the coupling procedure was changed: During the symmetrical anhydride couplings, 20% of 2,2,2-trifluoroethanol (hereinafter designated TFE) was added after 10 minutes of coupling. During the HOBt couplings 20% of TFE was added during the second coupling.

After completion of the synthesis, the Boc group was removed as mentioned above and 242 mg of the peptide resin was subjected to a cleavage and deprotection procedure using a mixture of hydrogen fluoride, anisole and methyl ethyl sulfide at 0°C as described in Schiller, P.W. et al.: Biochem.Biophys.Res.Com. 131 (1985), 1056 - 62. After evaporation of the hydrogen fluoride the liberated peptide was precipitated with diethyl ether and washed several times with ethyl acetate. Then the precipitate was extracted with 10 ml of 50% acetic acid and the extract was subjected to gel filtration in 2% acetic acid on a column of Sephadex G-25. The peptide containing fractions were collected and 9.7 mg of dithiothreitol was added. The pH of the solution was brought to 9.5 (ammonium hydroxide solution) and the solution was left for 1 hour at room temperature. Then the pH value was adjusted to 8.0 (acetic acid) and the resulting mixture was diluted to 120 ml (water). A solution of 138 mg of potassium ferricyanide in 10 ml of water was added with stirring during 2.5 hours. The pH value was then lowered further to 5.0 (acetic acid) and the solution was treated with 3 g of Lewatit MP 5080 ion exchanger (on $Cl^-$ form) for 0.5 hours. The ion exchanger was removed by filtration and the resulting colourless solution was lyophilized.

Purification

30 mg of the crude product (containing approximately 20% peptide) was dissolved in 10% acetic acid. After stirring at 40 - 45°C for 2 hours the solution was filtered (Millex-GV 0.22 µm) and subjected to chromatography on a Merck column (HIBAR, 10 x 250 mm LiChrosorb. RP-18, 7 µm). The column was eluted at 4 ml/min. with an acetonitrile/water mixture containing 0.1 M ammoniumacetate (pH 3.3). The initial

concentration of $CH_3CN$ was 15% and it was increased by a linear gradient to 45% during 30 minutes. Fractions containing the product were collected. To the combined collected fractions was added an equivalent amount of water and the solution obtained was concentrated to half volume (rotary evaporator). A corresponding volume of water was added and the product was lyophilized at -20°C. The product was characterized by amino acid analysis and HPLC analysis.

For the HPLC analysis a 4 x 200 mm column packed with 5 µ Nucleosil octadecyl derivatized silica (Machery Nagel, Düren, FRG. No. 72006) was used. The column was eluted at 1 ml/min. with an acetonitrile/0.1 M $Na_2SO_4$ mixture. The initial concentration of $CH_3CN$ was 15% and it was increased by a linear gradient to 35% during 25 minutes (A). Retention time: 22.12 minutes.

Example 2

ANP-(5-28)-Pro-$NMe_2$ was prepared by conventional peptide synthesis in solution in analogy with procedures described by M. Bodanszky: Principles of Peptide Synthesis (Springer-Verlag 1984) and found in The Peptides Vol. 1-5, Ed. E. Gross and J. Meienhofer (Academic Press 1979). In the synthesis of ANP-(5-28)-Pro-$NMe_2$, Boc, But, OBut, Acm and protonation (for Arg) were used for permanent protection, OMe was used for semipermanent protection and Z and Fmoc (in the presence of Cys) were used for temporary protection. The following five protected ANP-fragments:

A) Boc-[5-10]-OH,
B) Z-[11-13]-OH,
C) H-[14-16]-OH,
D) Fmoc-[17-22]-OH,
E) H-[23-28]-$Pro^{29}$-$NMe_2$, were prepared by coupling the protected amino acid to the C-terminal amino acid methyl ester by the mixed anhydride procedure using IOC-Cl, except for Z-Asn-OH where the hydroxysuccinimide ester was used. The methyl ester in the C-terminal was removed by saponification, the N-terminal Z-group by hydrogenolysis and the N-terminal Fmoc-group by treatment with piperidine.

The protected fragments were linked together by the mixed anhydride or the EDAC/HOBt methods. The fragments B and C were assembled to Z-[11-16]-OH which after removal of the Z-group by hydrogenolysis was coupled to fragment A affording Boc-[5-16]-OH. The fragments D and E were assembled to Fmoc-[17-28]-$Pro^{29}$-$NMe_2$ which after removal of the Fmoc-group was coupled to fragment Boc-[5-16]-OH by the EDAC/HOBt method. The protecting groups were cleaved from Boc-[5-28]-$Pro^{29}$-$NMe_2$ by TFA and the resulting [$Cys^7$(Acm),$Cys^{23}$(Acm)]ANP-(5-28)-$Pro^{29}$-$NMe_2$ was converted to ANP-(5-28)-$Pro^{29}$-$NMe_2$ by iodine in 80% acetic acid. The crude peptide was subjected to semipreparative HPLC purification as described in Example 1, yielding pure peptide with retention time: 19.62 minutes in a HPLC analysis as described in Example 1. The amino acid analysis was in agreement with the amino acid composition of the compound.

Example 3

The following compounds were prepared by procedures similar to those described in Example 1. For those prepared according to Example 1, either the MBHA-resin was used for peptides with a C-terminal carboxamide or the so-called PAM-resin (described by A.R. Mitchell et al.: J.Org.Chem. 43 (1978), 2845 - 52) was used for peptides with a C-terminal carboxylic acid. In most cases, the resins were acylated with a non amino acid in the last step to produce N-acylated peptides. The compounds were subjected to HPLC analysis either as described in Example 1 (which in Table I has been designated condition A) or by a slightly modified procedure where the initial concentration of $CH_3CN$ was 25% and the final concentration of $CH_3CN$ during the gradient was 45% (which in Table I has been designated condition B). For all compounds, amino acid analysis was in agreement with the amino acid composition of the compounds.

Table I

| COMPOUND | PREPARATION | | | HPLC ANALYSIS | |
|---|---|---|---|---|---|
| Structure | Code No. | Preparation according to Ex. No. | Resin type used | Retention time, minutes | Conditions |
| [$N^{\alpha}$-acetyl-$Arg^{4}$,D-$Ala^{9}$,$Leu^{18}$,D-$Ala^{20}$,D-$Ala^{22}$,N-$MePhe^{26}$,D-$Tyr^{28}$]ANP-(4-28),$Pro^{29}$-$NH_2$ | NO-70-0270 | 1 | MBHA | 25.15 | A |
| ANP-(5-28)-N-$MePhe^{29}$-$NH_2$ | NO-70-273 | 1 | MBHA | 22.35 | A |
| [N-(4-guanidinobenzoyl)-$Ser^{5}$]-ANP-(5-28)-N-$MePhe^{29}$-$NH_2$ | NO-70-0317 | 1 | MBHA | 9.16 | B |
| [N-(4-guanidinobutyryl)-$Ser^{5}$]-ANP-(5-28)-N-$MePhe^{29}$-$NH_2$ | NO-70-0337 | 1 | MBHA | 9.29 | B |
| [$N^{\alpha}$-acetyl-D-$Arg^{4}$,D-$Ala^{9}$,$Gln^{11}$,-$Leu^{18}$,D-$Ala^{20}$,D-$Ala^{22}$,N-$MePhe^{26}$,$Pro^{28}$]ANP-(4-28)-$Pro^{29}$-$NH_2$ | NO-70-0338 | 1 | MBHA | 12.54 | B |
| [$N^{\alpha}$-acetyl-D-$Arg^{4}$,D-$Ala^{9}$,$Gln^{11}$,-D-$Ala^{16}$,D-$Leu^{18}$,D-$Ala^{20}$,D-$Ala^{22}$,-$Ala^{25}$,N-$MePhe^{26}$,D-$Tyr^{28}$]ANP-(4-28)-$Pro^{29}$-$NH_2$ | NO-70-0355 | 1 | MBHA | 15.16 | B |

| | | | | | |
|---|---|---|---|---|---|
| [$Arg^{17}$,$Ile^{18}$]ANP-(5-28) | NO-70-0351 | 1 | Pam | 19.62 | A |
| [$N^{\alpha}$-acetyl-$Arg^{4}$,D-$Ala^{9}$,$Leu^{18}$,-D-$Ala^{20}$,D-$Ala^{22}$,N-$MePhe^{26}$,-D-$Tyr^{28}$]ANP-(4-28)-$Pro^{29}$-Asp-β-(NH-phenethyl)$^{30}$-$NH_2$ | NO-70-0352 | 1 | MBHA | 17.00 | B |
| [$N^{\alpha}$-acetyl-$Arg^{4}$,Orn(2-pyrimidinyl)$^{8}$,D-$Ala^{9}$,$Leu^{18}$,D-$Ala^{20}$,D-$Ala^{22}$,N-$MePhe^{26}$-D-$Tyr^{28}$]ANP-(4-28)-$Pro^{29}$-$NH_2$ | NO-70-0354 | 1 | MBHA | 11.00 | B |
| [$N^{\alpha}$-acetyl-$Arg^{4}$,D-$Ala^{9}$,$Leu^{18}$,-D-$Ala^{20}$,m-(NH-methyl)benzoyl$^{21-22}$,N-$MePhe^{26}$,D-$Tyr^{28}$]ANP-(4-28)-$Pro^{29}$-$NH_2$ | NO-70-0353 | 1 | MBHA | 9.33 | B |
| [$N^{\alpha}$-acetyl-$Arg^{4}$,D-$Ala^{9}$,$Leu^{18}$,D-$Ala^{20}$,D-$Ala^{22}$,N-$MePhe^{26}$,$Pro^{28}$]-ANP-(4-28)-$Pro^{29}$-$NH_2$ | NO-70-0362 | 1 | MBHA | 10.25 | B |
| [$N^{\alpha}$-acetyl-$Arg^{4}$,D-$Ala^{9}$,$Leu^{18}$,D-$Ala^{20}$,D-$Ala^{22}$,N-$MePhe^{26}$]ANP-(4-28)-$Phe^{29}$-$Gln^{30}$-$Tyr^{31}$-$Phe^{32}$-$NH_2$ | NO-70-0377 | 1 | MBHA | 17.45 | B |

| | | | | | |
|---|---|---|---|---|---|
| [$N^{\alpha}$-acetyl-$Arg^{4}$,D-$Ala^{9}$,$Leu^{18}$,-D-$Ala^{20}$,D-$Ala^{22}$,N-$MePhe^{26}$,D-$Tyr^{28}$]ANP-(4-28)-$Pro^{29}$,$Arg^{30}$,-$Pro^{31}$-$NH_2$ | NO-70-0378 | 1 | MBHA | 9.95 | B |
| [$N^{\alpha}$-acetyl-$Arg^{4}$,D-$Ala^{9}$,$Leu^{18}$,-D-$Ala^{20}$,D-$Ala^{22}$,N-$MePhe^{26}$,-D-$Tyr^{28}$]ANP-(4-28)-$Pro^{29}$-$Glu^{30}$-$NH_2$ | NO-70-0395 | 1 | MBHA | 11.50 | B |
| [$N^{\alpha}$-acetyl-$Arg^{4}$,D-$Ala^{9}$,$Leu^{18}$,-D-$Phe^{20}$,D-$Ala^{22}$,N-$MePhe^{26}$,-D-$Tyr^{28}$]ANP-(4-28)-$Pro^{29}$-$NH_2$ | NO-70-0392 | 1 | MBHA | 15.45 | B |
| [$N^{\alpha}$-acetyl-$Arg^{4}$,D-$Ala^{9}$,$Trp^{12}$,-$Leu^{18}$,D-$Ala^{20}$,D-$Ala^{22}$,N-$MePhe^{26}$,-D-$Tyr^{28}$]ANP-(4-28)-$Pro^{29}$-$NH_2$ | NO-70-0400 | 1 | MBHA | 12.97 | B |
| [$N^{\alpha}$-acetyl-$Arg^{4}$,m-NH-phenyl-$acetyl^{8-9}$,$Leu^{18}$,D-$Ala^{20}$,D-$Ala^{22}$,-N-$MePhe^{26}$,D-$Tyr^{28}$]ANP-(4-28)-$Pro^{29}$-$NH_2$ | NO-70-0410 | 1 | MBHA | 10.45 | B |

| | | | | | |
|---|---|---|---|---|---|
| [N$^{\alpha}$-acetyl-Arg$^{4}$,D-Ala$^{9}$,Asp$^{10}$,-Leu$^{18}$,D-Ala$^{20}$,D-Ala$^{22}$,N-MePhe$^{26}$,-D-Tyr$^{28}$]ANP-(4-28)-Pro$^{29}$-$NH_2$ | NO-70-0422 | 1 | MBHA | 11.04 | B |
| [N$^{\alpha}$-acetyl-Arg$^{4}$,m-(NH-methyl)-benzoyl$^{9-10}$,Leu$^{18}$,D-Ala$^{20}$,-D-Ala$^{22}$,N-MePhe$^{26}$,D-Tyr$^{28}$]-ANP-(4-28)-Pro$^{29}$-$NH_2$ | NO-70-0401 | 1 | MBHA | 12.10 | B |
| [N$^{\alpha}$-acetyl-Arg$^{4}$,Trp$^{8}$,D-Ala$^{9}$,-Leu$^{18}$,D-Ala$^{20}$,D-Ala$^{22}$,N-MePhe$^{26}$,-D-Tyr$^{28}$]ANP-(4-28)-Pro$^{29}$-$NH_2$ | NO-70-0397 | 1 | MBHA | 11.87 | B |
| [N$^{\alpha}$-acetyl-Arg$^{4}$,D-Ala$^{9}$,Leu$^{18}$,-D-Ala$^{20}$,D-Ala$^{22}$,N-MePhe$^{26}$,Trp$^{28}$]-ANP-(4-28)-Trp$^{29}$-$NH_2$ | NO-70-0389 | 1 | MBHA | 12.01 | B |
| [N$^{\alpha}$-acetyl-Arg$^{4}$,D-Trp$^{9}$,Leu$^{18}$,-D-Ala$^{20}$,D-Ala$^{22}$,N-MePhe$^{26}$,-D-Tyr$^{28}$]ANP-(4-28)-Pro$^{29}$-$NH_2$ | N-70-0390 | 1 | MBHA | 14.93 | B |
| [N$^{\alpha}$-acetyl-Arg$^{4}$,D-Ala$^{9}$,D-Leu$^{16}$,-Leu$^{18}$,D-Ala$^{20}$,D-Ala$^{22}$,N-MePhe$^{26}$,-D-Tyr$^{28}$]ANP-(4-28)-Pro$^{29}$-$NH_2$ | NO-70-0391 | 1 | MBHA | 16.32 | B |

| | | | | | |
|---|---|---|---|---|---|
| [$N^{\alpha}$-acetyl-$Arg^{4}$,D-$Ala^{9}$,$Leu^{18}$,-D-$Ala^{20}$,D-$Ala^{22}$,N-$MePhe^{26}$,-D-$Tyr^{28}$]ANP-(4-28)-$Pro^{29}$-$Gln^{30}$-$NH_2$ | NO-70-0405 | 1 | MBHA | 10.79 | B |
| [$N^{\alpha}$-acetyl-$Arg^{4}$,D-$Ala^{9}$,$Leu^{18}$,-D-$Ala^{20}$,D-$Ala^{22}$,N-$MePhe^{26}$]-ANP-(4-28)-$Pro^{29}$-$NH_2$ | NO-70-0404 | 1 | MBHA | 10.79 | B |
| [$N^{\alpha}$-acetyl-$Arg^{4}$,D-$Ala^{9}$,$Leu^{18}$,-D-$Ala^{20}$,$Trp^{21}$,D-$Ala^{22}$,N-$MePhe^{26}$,-D-$Tyr^{28}$]ANP-(4-28)-$Pro^{29}$-$NH_2$ | NO-70-0403 | 1 | MBHA | 11.59 | B |
| [$N^{\alpha}$-acetyl-$Arg^{4}$,D-$Ala^{9}$,$Gln^{11}$,-$Arg^{13}$,$Asp^{14}$,$Leu^{18}$,D-$Ala^{20}$,-D-$Ala^{22}$,N-$MePhe^{26}$,D-$Tyr^{28}$]-ANP-(4-28)-$Pro^{29}$-$NH_2$ | NO-70-0420 | 1 | MBHA | 12.34 | B |
| [$N^{\alpha}$-acetyl-$Arg^{4}$,D-$Ala^{9}$,$Leu^{18}$,-D-$Ala^{20}$,D-$Ala^{22}$,N-$MePhe^{26}$,-N-$MeArg^{27}$,D-$Tyr^{28}$]ANP-(4-28)-$Pro^{29}$-$NH_2$ | NO-70-0406 | 1 | MBHA | 10.21 | B |

| | | | | | |
|---|---|---|---|---|---|
| [$N^{\alpha}$-acetyl-$Arg^{4}$,D-$Phe^{9}$,D-$Ala^{16}$,-$Leu^{18}$,D-$Ala^{20}$,D-$Ala^{22}$,N-$MePhe^{26}$,-$Pro^{28}$]ANP-(4-28)-$Pro^{29}$-$NH_2$ | NO-70-0431 | 1 | MBHA | 27.50 | A |
| [$N^{\alpha}$-acetyl-$Arg^{4}$]ANP-(4-28)-$Phe^{29}$-$Gln^{30}$-$Tyr^{31}$-$Phe^{32}$-$NH_2$ | NO-70-0432 | 1 | MBHA | 24.62 | A |
| [$N^{\alpha}$-acetyl-$Arg^{4}$,$Pro^{28}$]ANP-(4-28)-$Pro^{29}$-$NH_2$ | NO-70-0433 | 1 | MBHA | 15.58 | A |
| [$N^{\alpha}$-acetyl-$Arg^{4}$,D-$Ala^{9}$,$Leu^{18}$,-D-$Ala^{22}$,N-$MePhe^{26}$,$Pro^{28}$]-ANP-(4-28)-$Pro^{29}$-$NH_2$ | NO-70-0542 | 1 | MBHA | 22.68 | A |
| [$N^{\alpha}$-acetyl-$Arg^{4}$,N-$MePhe^{26}$,$Pro^{28}$]-ANP-(4-28)-$Pro^{29}$-$NH_2$ | NO-70-0597 | 1 | MBHA | 16.79 | A |

Example 4

The following compounds were prepared as illustrated in Example 2, purified as described in Example 1 and analysed by HPLC as shown in Example 3.

ANP-(5-28)-$Pro^{29}$-NH-(1-adamantyl)

HPLC analysis (condition B) retention time: 15.00 minutes.

ANP-(5-28)-$Pro^{29}$-NH-lauryl

HPLC analysis (condition B) retention time: 26.00 minutes.

For both compounds amino acid analysis was in agreement with the amino composition of the compounds.

The feature disclosed in the foregoing description and in the following claims may, both separately and in any combination thereof, be material for realising the invention in diverse forms thereof.

**Claims**

1. Compounds of the general formula I

$R^a$-$Ser_{n1}$-$Leu_{n2}$-$Arg_{n3}$-$(R^4)_{n4}$-$(R^5)_{n5}$-$(R^6)_{n6}$-Cys-$R^8$-$R^9$-$R^{10}$-$R^{11}$-$R^{12}$-$R^{13}$-$R^{14}$-Ile-$R^{16}$-$R^{17}$-$R^{18}$-$R^{19}$-$R^{20}$-$R^{21}$-$R^{22}$-Cys-$R^{24}$-$R^{25}$-$R^{26}$-$R^{27}$-$R^{28}$-$(R^{29})_{n29}$-$(R^{30})_{n30}$-$(R^{31})_{n31}$-$(R^{32})_{n32}$-$(R^{33})_{n33}$-$(R^{34})_{n34}$-$(R^{35})_{n35}$-$(R^{36})_{n36}$-$(R^{37})_{n37}$ $(R^{38})_{n38}$-$R^b$

(I)

wherein $R^4$ is Arg, Lys or D-Arg, $R^5$ is Ser or Ile, $R^6$ is Ser, D-Ser or Asp, $R^8$ is Phe, Leu, N-Bzl-Gly, Orn(2-pyrimidinyl) or Trp, $R^9$ is Gly, D-Trp, D-Ala or D-Phe, $R^{10}$ is Gly, Asp or Pro, $R^{11}$ is Arg, Lys, Gln or Orn(2-pyrimidinyl), $R^{12}$ is Ile, Met or Trp, $R^{13}$ is Asp, Glu, Asn, Lys or Arg, $R^{14}$ is Arg, Lys, Orn(2-pyrimidinyl) or Asp, $R^{16}$ is Gly, D-Leu, D-Ala or D-Arg, preferably Gly, $R^{17}$ is Ala, Leu, Ile, Lys or Arg, $R^{18}$ is Gln, Arg, Lys, Leu, D-Leu or Ile, $R^{19}$ is Ser, Ile, Ala or Asp, $R^{20}$ is Gly, D-Ala, Val, D-Phe, Lys or Arg, $R^{21}$ is Leu, Ile or Trp, $R^{22}$ is Gly, D-Ala, Sar, D-Phe or Ala, $R^{24}$ is Asn, Pro, N-MeArg or N-MeAsn, $R^{25}$ is Ser, D-Ser or Ala, $R^{26}$ is Phe, D-Phe, N-MePhe, N-Bzl-Gly or Ile, $R^{27}$ is Arg, Lys, D-Arg, Orn(2-pyrimidinyl) or N-MeArg, $R^{28}$ is Tyr, D-Tyr, Pro or Trp, $R^{29}$ is Pro, Trp, Phe or N-MePhe, $R^{30}$ is Gln, Arg, Lys, Glu, Asp-$\beta$-(NH-phenethyl), Ala, Asn or Phe, $R^{31}$ is Tyr, Pro, Ser or Trp, $R^{32}$ is Phe, Leu or Tyr, $R^{33}$ is Gln, Glu, Leu or Arg, $R^{34}$ is Ser, Glu or Phe, $R^{35}$ is Phe, Ser or Leu, $R^{36}$ is Leu or Phe, $R^{37}$ is Arg or Gln, $R^{38}$ is Ser or Arg, or $R^8$ together with $R^9$ represents a group of the formula $-NH-m-C_6H_4-CH_2CO-$, $R^9$ together with $R^{10}$ represents a group of the formula $-NH-CH_2-m-C_6H_4-CO-$, $R^{21}$ together with $R^{22}$ represents a group of the formula $-NH-CH_2-m-C_6H_4-CO-$, n1, n2, n3, n4, n5, n6, n29, n30, n31, n32, n33, n34, n35, n36, n37 and n38 are the same or different and each represents zero or one, $R^a$ is hydrogen or acyl, and $R^b$ is hydroxy, amino or substituted amino such as alkylamino, N,N-dialkylamino, adamantylamino or aralkylamino such as phenethylamino, and wherein there is a disulfide bridge between $Cys^7$ and $Cys^{23}$, with the proviso that either at least two of the amino acid residues $R^8$, $R^9$, $R^{10}$, $R^{11}$, $R^{12}$, $R^{13}$, $R^{14}$, $R^{16}$, $R^{17}$, $R^{18}$, $R^{19}$, $R^{20}$, $R^{21}$ and $R^{22}$ are different from Phe, Gly, Gly, Arg, Ile or Met, Asp, Arg, Gly, Ala, Gln, Ser, Gly, Leu and Gly, respectively, or if the amino acid residue $R^{12}$ is Ile or Met, and the amino acid residues $R^8$, $R^9$, $R^{10}$, $R^{11}$, $R^{13}$, $R^{14}$, $R^{16}$, $R^{17}$, $R^{18}$, $R^{19}$, $R^{20}$, $R^{21}$ and $R^{22}$ are Phe, Gly, Gly, Arg, Asp, Arg, Gly, Ala, Gln, Ser, Gly, Leu and Gly, respectively, then n29 is one, and salts thereof.

2. Compounds, according to Claim 1, wherein at least three, preferably four, of the amino acid residues $R^8$, $R^9$, $R^{10}$, $R^{11}$, $R^{12}$, $R^{13}$, $R^{14}$, $R^{16}$, $R^{17}$, $R^{18}$, $R^{19}$, $R^{20}$, $R^{21}$ and $R^{22}$ are different from Phe, Gly, Gly, Arg, Ile or Met, Asp, Arg, Gly, Ala, Gln, Ser, Gly, Leu and Gly, respectively.

3. Compounds, according to Claim 1, having an amino acid sequence which differs from the amino acid sequence of ANP and h-ANP in at least 3 positions, preferably at least 4 positions, more preferred at least 5 positions, even more preferred at least 6 positions.

4. Compounds, according to Claim 1, wherein the amino acid residue $R^{12}$ is Ile or Met, the amino acid residues $R^8$, $R^9$, $R^{10}$, $R^{11}$, $R^{13}$, $R^{14}$, $R^{16}$, $R^{17}$, $R^{18}$, $R^{19}$, $R^{20}$, $R^{21}$ and $R^{22}$ are Phe, Gly, Gly, Arg, Asp, Arg, Gly, Ala, Gln, Ser, Gly, Leu and Gly, respectively, and n29 is one.

5. A process for preparing compounds of the general formula I stated in Claim 1 with the definitions stated in Claim 1, characterized by removing the protecting group(s) from a compound of the general formula II

$$R'^{a}-(R^{1})_{n1}-(R^{2})_{n2}-(R^{3})_{n3}-(R'^{4})_{n4}-(R'^{5})_{n5}-(R'^{6})_{n6}-R^{7}-R'^{8}-R'^{9}-R'^{10}-R'^{11}-R'^{12}-R'^{13}-R'^{14}-R^{15}-R'^{16}-R'^{17}-R'^{18}-R'^{19}-R'^{20}-R'^{21}-R'^{22}-R^{23}-R'^{24}-R'^{25}-R'^{26}-R'^{27}-R'^{28}-(R'^{29})_{n29}-(R'^{30})_{n30}-(R'^{31})_{n31}-(R'^{32})_{n32}-(R'^{33})_{n33}-(R'^{34})_{n34}-(R'^{35})_{n35}-(R'^{36})_{n36}-(R'^{37})_{n37}-(R'^{38})_{n38}-R'^{b} \quad (II)$$

wherein $R^{1}$ represents optionally substituted Ser, $R^{2}$ represents Leu, $R^{3}$represents optionally substituted Arg, $R^{15}$ represents Ile, $R^{7}$ and $R^{23}$ are the same or different and each represents optionally substituted Cys, $R'^{4}$, $R'^{5}$, $R'^{6}$, $R'^{8}$, $R'^{9}$, $R'^{10}$, $R'^{11}$, $R'^{12}$, $R'^{13}$, $R'^{14}$, $R'^{16}$ , $R'^{17}$, $R'^{18}$, $R'^{19}$, $R'^{20}$, $R'^{21}$, $R'^{22}$, $R'^{24}$ , $R'^{25}$, $R'^{26}$, $R'^{27}$, $R'^{28}$, $R'^{29}$, $R'^{30}$, $R'^{31}$ , $R'^{32}$, $R'^{33}$, $R'^{34}$, $R'^{35}$, $R'^{36}$, $R'^{37}$ and $R'^{38}$ are the same as $R^{4}$, $R^{5}$, $R^{6}$, $R^{8}$, $R^{9}$, $R^{10}$, $R^{11}$, $R^{12}$, $R^{13}$, $R^{14}$, $R^{16}$, $R^{17}$, $R^{18}$, $R^{19}$, $R^{20}$, $R^{21}$, $R^{22}$, $R^{24}$, $R^{25}$, $R^{26}$, $R^{27}$, $R^{28}$, $R^{29}$, $R^{30}$, $R^{31}$, $R^{32}$, $R^{33}$, $R^{34}$, $R^{35}$, $R^{36}$, $R^{37}$, $R^{38}$, respectively, or represents a protected amino acid residue wherein the parent amino acid residue is one of the amino acids mentioned for $R^{4}$, $R^{5}$, $R^{6}$, $R^{8}$, $R^{9}$, $R^{10}$, $R^{11}$, $R^{12}$, $R^{13}$, $R^{14}$, $R^{16}$, $R^{17}$, $R^{18}$, $R^{19}$, $R^{20}$, $R^{21}$, $R^{22}$, $R^{24}$, $R^{25}$, $R^{26}$, $R^{27}$, $R^{28}$, $R^{29}$, $R^{30}$, $R^{31}$, $R^{32}$, $R^{33}$, $R^{34}$, $R^{35}$, $R^{36}$, $R^{37}$ and $R^{38}$, respectively, or $R'^{8}$ together with $R'^{9}$ represents a group of the formula $-NH-m-CH_6H_4-CH_2CO-$, $R'^{9}$ together with $R'^{10}$ reprsents a group of the formula $-NH-CH-m-C_6H_4-CO-$, $R'^{21}$ together with $R'^{22}$ represents a group of the formula $-NH-CH_2-m-C_6H_4-CO$, nl, n2, n3, n4, n5, n6, n29, n30, n31, n32, n33, n34, n35, n36, n37 and n38 are the same of different and each represents zero or one, $R'^{a}$ is hydrogen, acyl or a protecting group, and $R'^{b}$ is optionally protected hydroxy, amino or substituted amino such as alkylamino, N,N-dialkylamino, adamantylamino or aralkylamino such as phenethylamino, and wherein there is a disulfide bridge between $Cys^{7}$ and $Cys^{23}$, with the proviso that either at least two of the amino acid residues $R^{8}$, $R^{9}$, $R^{10}$, $R^{11}$, $R^{12}$, $R^{13}$, $R^{14}$, $R^{16}$, $R^{17}$, $R^{18}$, $R^{19}$, $R^{20}$, $R^{21}$ and $R^{22}$ are different from Phe, Gly, Gly, Arg, Ile and Met, Asp, Arg, Gly, Ala, Gln, Ser, Gly, Leu and Gly, respectively, or if the amino acid residue $R^{12}$ is Ile or Met, and the amino acid residues $R^{8}$, $R^{9}$, $R^{10}$, $R^{11}$, $R^{13}$, $R^{14}$, $R^{16}$, $R^{17}$, $R^{18}$, $R^{19}$, $R^{20}$, $R^{21}$ and $R^{22}$ are Phe , Gly, Gly, Arg, Asp, Arg, Gly, Ala, Gln, Ser, Gly, Leu and Gly, respectively, then n29 is one, with the further proviso that at least one of the symbols $R'^{a}$, $R^{1}$, $R^{2}$, $R^{3}$, $R'^{4}$, $R'^{5}$ , $R'^{6}$, $R^{7}$, $R'^{8}$, $R'^{9}$, $R'^{10}$, $R'^{11}$, $R'^{12}$, $R'^{13}$ , $R'^{14}$, $R^{15}$, $R'^{16}$, $R'^{17}$, $R'^{18}$, $R'^{19}$, $R'^{20}$, $R'^{21}$ , $R'^{22}$, $R^{23}$, $R'^{24}$, $R'^{25}$, $R'^{26}$, $R'^{27}$, $R'^{28}$, $R'^{29}$ , $R'^{30}$, $R'^{31}$, $R'^{32}$, $R'^{33}$, $R'^{34}$, $R'^{35}$, $R'^{36}$ , $R'^{37}$, $R'^{38}$ and $R'^{b}$ is different from $R^{a}$, Ser, Leu, Arg, $R^{4}$, $R^{5}$, $R^{6}$, Cys, $R^{8}$, $R^{9}$, $R^{10}$, $R^{11}$, $R^{12}$, $R^{13}$, $R^{14}$, Ile, $R^{16}$, $R^{17}$, $R^{18}$, $R^{19}$, $R^{20}$, $R^{21}$, $R^{22}$, Cys, $R^{24}$, $R^{25}$, $R^{26}$, $R^{27}$, $R^{28}$, $R^{29}$, $R^{30}$, $R^{31}$, $R^{32}$, $R^{33}$, $R^{34}$, $R^{35}$, $R^{36}$, $R^{37}$, $R^{38}$ and $R^{b}$, respectively, or a salt thereof, preferably by acidolysis and, if necessary, oxidizing the compound prepared, and, if desired, converting a compound of a formula I into a salt thereof or converting a salt of a compound of formula I into a compound of formula I.

6. Process, according to Claim 5, characterized in that in the compound of formula II the guanidino groups of the arginines and the N-methylarginines are optionally protected by protonation or by a protection group selected from the group consisting of benzenesulfonyl substituted by lower alkyl and/or lower alkoxy (e.g. tosyl, 4-methoxy-2,3,6-trimethylbenzenesulfonyl or 2,4,6-trimethylbenzenesulfonyl), adamantyloxycarbonyl, benzyloxycarbonyl and nitro, the hydroxy groups of the serines are optionally protected using a protection selected from the group consisting of lower tert.alkyl (e.g. tert.butyl or tert.amyl), benzyl, clorine or bromine substituted benzyl, lower sec.alkyl (e.g. cyclohexyl or cyclopentyl) and benzyl substituted by lower alkyl and/or lower alkoxy (e.g. 4-methoxybenzyl), the indole moities of the tryptophanes are optionally protected using a protection selected from the group consisting of formyl and benzenesulfonyl substituted by lower alkyl and/or lower alkoxy (e.g. 4-methoxy-2,3,6-trimethylbenzenesulfonyl or 2,4,6-trimethylbenzenesulfonyl), the side chain carboxyl moities of glutamic acids and aspartic acids are optionally protected using a protection selected from the group consisting of lower tert.alkyl ester (e.g. tert.butyl or tert.amyl), lower sec.alkyl ester (e.g. cyclohexyl ester or cyclopentyl ester), benzyl ester, lower alkyloxy substituted benzylester (e.g. 4-methoxybenzyl ester) and clorine or bromine substituted benzyl ester (e.g. 4-chlorobenzyl ester or 4-bromobenzyl ester), the side chain amino moities of lysines are optionally protected using a protection selected from the group consisting of lower tert.alkyloxycarbonyl (e.g. tert.butyloxycarbonyl or tert.amyloxycarbonyl), benzyloxycarbonyl, clorine, bromine or nitro substituted benzyloxycarbonyl (e.g. 4-nitrobenzyloxycarbonyl, 2-chlorobenzyloxycarbonyl or 2-bromobenzyloxycarbonyl), lower alkoxy substituted benzyloxycarbonyl (e.g. 4-methoxybenzyloxycarbonyl) and benzenesulfonyl substituted by lower alkyl and/or lower alkoxy (e.g. 4-methoxy-2,3,6-trimethylbenzenesulfonyl), the side chain amido moities of asparagines, glutamines and N-methylasparagines are optionally protected using a protection selected from the group consisting of xantyl, lower alkyloxy substituted benzyl (e.g. 4-methoxybenzyl or 2,4-dimethoxybenzyl), diphenylmethyl, lower alkyl substituted diphenylmethyl (e.g. 4,4'-dimethyldiphenylmethyl) and lower alkyloxy substituted diphenylmethyl (e.g. 4,4'-dimethoxydiphenylmethyl), the thioether moity of methionines are optionally protected as the corresponding sulfoxides, the residues in position 7 and 23 are either cystines linked together by a disulfide bridge or they are cysteines in which the sulfhydryl groups are protected using a protection selected from the group consisting of lower alkyl substituted benzyl (e.g. 4-methylbenzyl), lower alkyloxy substituted benzyl (e.g. 4-methoxybenzyl), triphenylmethyl, acetamidomethyl,

lower tert.alkyl (e.g. tert.butyl), diphenylmethyl, lower alkyloxy substituted diphenylmethyl (e.g. 4,4'-dimethoxydiphenylmethyl) and lower alkyl mercapto (e.g. tert.butyl mercapto or ethyl mercapto), R'a may be a protection group selected from the group as defined for the protection of the side chains of the lysines, and R'b may be a protection group selected from the group as defined for the protection of the side chains of the aspartic acids and glutamic acids and furthermore can be a polymer linked ester (e.g. oxymethylcopoly(styrenedivinylbenzene), 4-(oxymethyl)-phenylacetamidomethylcopoly(styrene-divinylbenzene), 4-(oxymethyl)phenoxymethylcopoly(styrene-divinylbenzene) or 4-(oxymethyl)benzoylaminomethylcopoly(styrene-divinylbenzene)) or R'b may be an alkylamino group where the alkyl moity is selected from the group as defined for the protection of the side chains of the asparagines and glutamines and furthermore the alkyl may be a polymer linked alkyl (e.g. 4-(copoly(styrene-divinylbenzene))-diphenylmethyl, 4-(copoly(styrene-divinylbenzene))-4'methyldiphenylmethyl or 4-(5-oxy-n-pentanoylaminomethylcopoly(styrene-divinylbenzene))-2,6-dimethoxybenzyl), characterized by an acidolytic reaction in which the protected compounds are treated with an acid such as hydrogen fluoride, trifluoroacetic acid, trifluoromethanesulfonic acid, methanesulfonic acid or mixtures thereof in the presence of proper additives, and after the acidolytic reaction a polymer linked protecting group which still may be attached to the target compound is cleaved in a separate reaction step (e.g. acidolysis, ammololysis or base treatment) and, if necessary, the process is followed by an oxidative reaction using e.g. iodine, oxygen or ferricyanide ions to form the disulfide bridge between residue 7 and 23.

7. A pharmaceutical composition comprising one or more compounds in accordance with Claim 1 or a pharmacologically acceptable salt thereof and a physiologically acceptable carrier or diluent.

8. One or more compounds in accordance with Claim 1 for use as a medicament.

9. One or more compounds in accordance with Claim 1 for inducing natriuresis, diuresis or vasodilation.

10. One or more compounds in accordance with Claim 1 for treatment of oedematous states, such as congestive heart failure, nephrotic syndrome and hepatic cirrhosis, or of hypertension or renal failure due to ineffective renal perfusion or reduced glomerular filtration rate.

11. The use of one or more compounds in accordance with Claim 1 for preparing antisera for use in immunoassay employing labelled reagents.